(19) **Europäisches Patentamt**

**European Patent Office**

**Office européen des brevets**

(11) Veröffentlichungsnummer : **0 460 128 B1**

(12)                              **EUROPÄISCHE PATENTSCHRIFT**

(45) Veröffentlichungstag der Patentschrift :
09.06.93 Patentblatt 93/23

(51) Int. Cl.⁵ : **A61K 7/13**

(21) Anmeldenummer : **90916740.5**

(22) Anmeldetag : **20.10.90**

(86) Internationale Anmeldenummer :
**PCT/EP90/01789**

(87) Internationale Veröffentlichungsnummer :
**WO 91/09587 11.07.91 Gazette 91/15**

(54) **MITTEL UND VERFAHREN ZUM OXIDATIVEN FÄRBEN VON HAAREN.**

(30) Priorität : **21.12.89 DE 3942294**

(43) Veröffentlichungstag der Anmeldung :
**11.12.91 Patentblatt 91/50**

(45) Bekanntmachung des Hinweises auf die
Patenterteilung :
**09.06.93 Patentblatt 93/23**

(84) Benannte Vertragsstaaten :
**DE ES FR GB IT**

(56) Entgegenhaltungen :
**EP-A- 0 376 047**

(56) Entgegenhaltungen :
**DE-A- 3 834 142**
**FR-A- 2 180 651**
**FR-A- 2 540 106**
**GB-A- 486 086**

(73) Patentinhaber : **Wella Aktiengesellschaft**
**Berliner Allee 65**
**W-6100 Darmstadt (DE)**

(72) Erfinder : **MAGER, Herbert**
**Route du Roule 21**
**CH-1723 Marly (CH)**
Erfinder : **CLAUSEN, Thomas**
**Ernst-Pasqué-Strasse 35A**
**W-6146 Alsbach (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Gegenstand der Erfindung ist ein Mittel zum oxidativen Färben von Haaren auf der Basis einer in der Haarfärbung üblichen Entwicklersubstanz-Kupplerstubstanz-Kombination, welches einen Gehalt an 2-Amino-6-chlor-4-nitrophenol aufweist.

In der heutigen Haarfärbepraxis haben Oxidationshaarfärbemittel eine wesentliche Bedeutung erlangt. Oxidationshaarfärbemittel enthalten Entwickler- und Kupplersubstanzen, wobei durch oxidative Kupplung mit Hilfe eines geeigneten Oxidationsmittels die Färbung im Haarschaft erzeugt wird.

An Oxidationsfarbstoffe, die zur Färbung menschlicher Haare verwendet werden, sind zahlreiche besondere Anforderungen gestellt. Die Farbstoffe müssen in toxikologischer und dermatologischer Hinsicht unbedenklich sein und Färbungen in der gewünschten Intensität ermöglichen. Ferner wird für die erzielten Haarfärbungen eine gute licht-, Dauerwell-, Säure- und Reibechtheit gefordert.

Neben den Farbstoffvorstufen und dem Oxidationsmittel enthalten Oxidationshaarfärbemittel Alkali, üblicherweise Ammoniak. Das Alkali bewirkt die Quellung des Haares und beschleunigt so die Penetration der Farbstoffvorstufen in den Haarschaft.

Durch die Kombination geeigneter Kuppler- und Entwicklersubstanzen lassen sich unterschiedliche Farbnuancen erzeugen. Zur Erzeugung von Naturtönen werden bevorzugt Resorcin, 4-Chlorresorcin, 4-Amino-1,2-methylendioxybenzol, 4-Hydroxy-1,2-methylendioxybenzol, 4-(2'-Hydroxyethylamino)-1,2-methylendioxybenzol, 2-Methylresorcin und m-Aminophenol in Kombination mit Entwicklersubstanzen wie 2,5-Diaminotoluol eingesetzt.

FR-A-2180651 offenbart substituierte 2-Amino 4-Nitrophenole, wobei die Aminogruppe stehs weiter substituiert ist und der Ring in 5 oder 6 -Stellung unter anderen möglichen Substituenten auch Cl trägen kann.

Bei der Nuancierung der Naturtöne auf rein oxidativer Basis wird aber festgestellt, daß diese, insbesondere bei helleren Farbtönen, auf natürlichem, grauem Haar durchsichtig und leer wirken.

Eine Möglichkeit, das Deck- und Ausgleichsvermögen von Oxidationshaarfärbemitteln zu verbessern, ist die Erhöhung der Alkalimenge, da dies die Penetration des Färbemittels verbessert und damit den Farbausgleich unterstützt. Andererseits führt die Erhöhung der Alkalimenge zu einer Verstärkung der oxidativen Schädigung der Haare, die nicht erwünscht ist.

Es bestand daher die Aufgabe, ein Oxidationshaarfärbemittel zu entwickeln, das ein verbessertes Deck- und Ausgleichsvermögen insbesondere bei helleren Farbtönen zeigt, ohne durch einen erhöhten Alkaligehalt die oxidative Schädigung der Haare zu verstärken.

Hierzu wurde nun gefunden, daß ein Mittel zum oxidativen Färben von Haaren auf der Basis einer in der Haarfärbung üblichen Entwicklersubstanz-Kupplersubstanz-Kombination, welches 0,01 bis 1,0 Gewichtsprozent 2-Amino-6-chlor-4-nitrophenol oder dessen physiologisch verträgliches, wasserlösliches Salz enthält, die gestellte Aufgabe in hervorragender Weise löst.

Durch den Gehalt an 2-Amino-6-chlor-4-nitrophenol werden die Deckkraft und das Ausgleichsvermögen des Oxidationshaarfärbemittels deutlich verbessert. Gleichzeitig ist es möglich, den Gehalt an Alkali in dem Mittel zu senken, was, insbesondere bei regelmäßiger Anwendung des Haarfärbemittels über einen längeren Zeitraum, zu einer deutlich geringeren Haarschädigung führt.

Ein weiterer Vorteil des beschriebenen Oxidationshaarfärbemittels ist die außerordentlich gute Verträglichkeit des Mittels in physiologischer Hinsicht. Das 2-Amino-6-chlor-4-nitrophenol ist ein vollständig unschädlicher Farbstoff, so daß bei geeigneter Auswahl der Oxidationshaarfarbstoffe besonders gut verträgliche Haarfärbemittel hergestellt werden können. Dies ist außerordentlich überraschend, da der chlorfreie Grundkörper, das 2-Amino-4-nitrophenol, gerade aufgrund seiner schlechten toxikologischen Eigenschaften nicht verwendet werden kann und die Verbesserung dieser Eigenschaften durch Einführung eines Chloratoms nicht zu erwarten war.

Völlig überraschend ist zudem die hohe Stabilität des 2-Amino-6-chlor-4-nitrophenols in Oxidationshaarfärbemitteln. Diese enthalten üblicherweise Reduktionsmittel wie Sulfit oder Ascorbinsäure, um die oxidationsempfindlichen Farbstoffvorstufen vor dem Sauerstoff der Luft bei Herstellung, Abfüllung, Lagerung und Verarbeitung zu schützen. Die Haarfärbemittel müssen über einen längeren Zeitraum, üblicherweise mehrere Jahre, in der Verpackung lagerstabil sein. Wird eine der Farbkomponenten während der Lagerzeit abgebaut, so verschiebt sich der Farbton bei der Ausfärbung in Abhängigkeit vom Alter des Färbemittels, was für ein verkaufsfähiges Produkt nicht akzeptabel ist. Diese Voraussetzung der Lagerstabilität wird aber häufig von Nitrofarbstoffen nicht erfüllt, da die Nitrogruppe aufgrund ihrer Struktur von Reduktionsmitteln angegriffen wird. Aus diesem Grunde sind viele Nitrofarbstoffe in Oxidationshaarfärbemitteln instabil oder aber werden im Verlaufe der Lagerzeit kontinuierlich abgebaut. Ein Verzicht auf das Reduktionsmittel verbessert zwar die Stabilität der Nitrofarbstoffe, das Färbemittel insgesamt wird aber anfällig gegen Luftsauerstoff.

Das 2-Amino-6-chlor-4-nitrophenol ist während der üblichen Lagerzeit von Oxidationshaarfärbemitteln vollstandig stabil und ermöglicht damit die Herstellung lagerstabiler, sehr gut deckender Haarfärbemittel, welche darüber hinaus zu einer verringerter Haarschädigung führen. Aus dem orangen Farbton der Nitroverbindung ergibt sich weiterhin, neben der Verbesserung der Deckkraft, eine leichte Verschiebung des Toncharakters nach Goldblond beziehungsweise Goldbraun, was zu sehr natürlich wirkenden Färbungen führt.

Aufgrund der, im Vergleich zum nicht chlorierten Grundkörper, sogar höheren Farbintensität sind relativ geringe Mengen des 2-Amino-6-chlor-4-nitrophenols für eine intensive, deckende Färbung bereits ausreichend. Vorzugsweise enthält das erfindungsgemäße Oxidationshaarfärbemittel 0,05 bis 0,3 Gewichtsprozent des 2-Amino-6-chlor-4-nitrophenols.

Von den in der Haarfärbung üblichen Kuppler- und Entwicklersubstanzen können in dem erfindungsgemäßen Mittel Resorcin, 4-Chlorresorcin, 4,6-Dichlorresorcin, 2-Methylresorcin, 2-Amino-4-(2'-hydroxyethylamino)-anisol, 2,4-Diaminobenzylalkohol, m-Phenylendiamin, 5-Amino-2-methylphenol, 2,4-Diaminophenoxyethanol, 1-Naphthol, m-Aminophenol, 3-Amino-4-chlor-6-methylphenol, 3-Amino-2-methylphenol, 4-Amino-2-hydroxyphenoxyethanol, 4-Hydroxy-1,2-methylendioxybenzol, 4-(2'-Hydroxyethylamino)-1,2-methylendioxybenzol, 2,4-Diamino-5-ethoxytoluol, 4-Hydroxyindol, 3-Amino-5-hydroxy-2,6-dimethoxypyridin und 3,5-Diamino-2,6-dimethoxypyridin, 2,6-Dimethoxypyridin, 2,5-Diaminotoluol, 2,5-Diaminobenzylalkohol, 2-(2',5'-Diaminophenyl)ethanol, 4-Aminophenol, 4-Amino-3-methylphenol, 4-Amino-2-methoxymethylphenol, 4-Amino-2-ethoxymethylphenol sowie Tetraaminopyrimidin oder deren physiologisch verträgliche, wasserlösliche Salze enthalten sein. Die Kuppler-und Entwicklersubstanzen können in den Oxidationshaarfärbemitteln jeweils einzeln oder im Gemisch miteinander enthalten sein.

Besonders bevorzugt enthält das erfindungsgemäße Oxidationshaarfärbemittel als Entwicklersubstanz 2-(2',5'-Diaminophenyl)ethanol oder dessen physiolgisch verträgliches Salz und mindestens eine Kupplersubstanz, die ausgewählt ist aus Resorcin, 2-Methylresorcin, 4-Chlorresorcin, 3,4-Methylendioxypnenol, m-Aminophenol und N-(2'-Hydroxyethyl)-3,4-methylendioxyanilin oder deren physiologisch verträglichen, wasserlöslichen Salzen.

Diese besonders bevorzugte Ausführungsform des erfindungsgemäßen Mittels ist ein besonders gut physiologisch verträgliches Oxidationshaarfärbemittel, das die Kombination einer nicht sensibilisierenden, nicht mutagenen Entwicklersubstanz-Kupplersubstanz-Kombination mit dem physiologisch gut verträglichen, ebenfalls nicht sensibilisierenden 2-Amino-6-chlor-4-nitrophenol enthält.

Die Gesamtmenge der in dem hier beschriebenen Oxidationshaarfärbemittel enthaltenen Entwicklersubstanz-Kupplersubstanz-Kombination soll 0,01 bis 6,0, vorzugsweise 0,1 bis 4,0, Gewichtsprozent betragen.

Die Entwicklerkomponenten werden im allgemeinen in etwa äqumolaren Mengen, bezogen auf die Kupplerkomponenten, eingesetzt. Es ist jedoch nicht nachteilig, wenn die Entwicklerkomponente diesbezüglich einen gewissen Unterschuß oder Überschuß vorhanden ist.

Weiterhin können die Oxidationshaarfärbemittel dieser Anmeldung zusätzlich andere Farbkomponenten, beispielsweise die mit sich selbst kuppelnden Farbstoffe 6-Amino-2-methylphenol, 2-Amino-5-methylphenol und 6-Amino-3-ethoxyphenol, sowie ferner übliche direktfärbenden Farbstoffe, zum Beispiel Triphenylmethanfarbstoffe wie Diamond Fuchsin (C.I. 42510) Leather Ruby HF (C.I. 42520), aromatische Nitrofarbstoffe wie 2-Amino-4,6-dinitrobenzol, 2-Amino-5-(2'-hydroxyethylamino)-nitrobenzol, 4-(2'-Hydroxyethylamino)-3-nitrotoluol, 1-(2'-Ureidoethylamino)-4-nitrobenzol und 2-Amino-5-nitrophenol, Azofarbstoffe wie Acid Brown 4 (C.I. 14805) und außerdem 1,4,5,8-Tetraaminoanthrachinon und 1,4-Diaminoanthrachinon enthalten. Die Haarfärbemittel können diese direktfärbenden und mit sich selbst kuppelnden Farbkomponenten in einer Menge von 0,1 - 4,0 Gewichtsprozent enthalten. Weitere geeignete direkt auf das Haar aufziehende Farbstoffe sind beispielsweise bei J.C. Johnson "Hair Dyes", Noyes Data Corp. Park Ridge, USA (1973), Seiten 3 - 91 und 113 - 139 (ISBN: 0-8155-0477-2) beschrieben.

Selbstverständlich können die Kuppler- und Entwicklersubstanzen sowie die anderen Farbkomponenten, sofern es Basen sind, auch in Form ihrer physiologisch verträglichen, wasserlöslichen Salze, beispielsweise als Hydrochlorid, Acetat oder Sulfat eingesetzt werden.

Darüberhinaus können in dem erfindungsgemäßen Oxidationshaarfärbemittel noch weitere übliche kosmetische Zusätze, beispielsweise Antioxidatien wie Ascorbinsäure, Thioglykolsäure oder Natriumsulfit, Parfümöle, Komplexbildner, Netzmittel, Emulgatoren, Verdicker, Pflegestoffe und andere vorhanden sein.

Die Zubereitungsform des Oxidationshaarfärbemittels kann die einer Lösung, vorzugsweise einer Creme, eines Gels oder einer Emulsion sein. Ihre Zusammensetzung stellt eine Mischung der Farbstoffkomponenten mit den für solche Zubereitungen üblichen Bestandteile dar. Als übliche Bestandteile von Cremes, Emulsionen oder Gelen kommen beispielsweise Netzmittel oder Emulgatoren aus den Klassen der anionischen, kationischen oder nichtioniogenen oberflächenaktiven Substanzen wie Fettalkoholsulfate, Alkylsulfonate, Alkylbenzolsulfonate, Alkyltrimethylammoniumsalze, oxethylierte Fettalkohole, oxethylierte Nonylphenole, Fettsäurealkanolamide, ferner Verdicker wie zum Beispiel höhere Fettalkohole, Stärke, Cellulosederivate, Paraffinöl und Fettsäuren sowie außerdem Pflegestoffe wie zum Beispiel Lanolinderivate, Cholesterin und Pantothensäure in Betracht. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, während die Verdicker in einer Menge von etwa 0,1 bis 25 Gewichtsprozent und die Pflegestoffe in einer Konzentration von 0,1 bis 5,0 Gewichtsprozent in den Zubereitungen enthalten sein können.

Je nach Zusammensetzung kann das erfindungsgemäße Oxidationshaarfärbemittel schwach sauer, neutral oder alkalisch reagieren. Insbesondere weist es einen pH-Wert im alkalischen Bereich zwischen 8,0 und 11,5 auf, wobei die Einstellung mit einem geeigneten Alkali, vorzugsweise mit Ammoniak, erfolgt. Das erfindungsgemäße Oxidationshaarfärbemittel enthält 0,01 bis 2,50 Gewichtsprozent, vorzugsweise 0,5 bis 2,00 Gewichtsprozent Ammoniak. Es können jedoch auch organische Amine, zum Beispiel Monoethanolamin oder Triethanolamin, Verwendung finden.

Bei dem erfindungsgemäßen Verfahren zur oxidativen Färbung von Haaren vermischt man das erfindungsgemäßen Oxidationshaarfärbemittel kurz vor dem Gebrauch mit einem Oxidationsmittel und trägt 60 bis 200 g dieses Gemisch auf das Haar auf. Als Oxidationsmittel zur Entwicklung der Haarfärbung kommen hauptsächlich Wasserstoffperoxid oder dessen Additionsverbindungen an Harnstoff, Melamin oder Natriumborat in Form der 3 bis 12prozentigen, vorzugsweise 6prozentigen, wäßrigen Lösungen in Betracht. Wird eine 6prozentige Wasserstoffperoxidlösung als Oxidationsmittel verwendet, so beträgt das Gewichtsverhältnis zwischen Haarfärbemittel und Oxidationsmittel 5:1 bis 1:2, vorzugsweise jedoch 1:1. Größere Mengen an Oxidationsmittel werden vor allem bei höheren Farbstoffkonzentrationen in Haarfärbemittel oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet. Gegebenenfalls wird im Anschluß an die Spülung mit einem Shampoo gewaschen und eventuell mit einer schwachen organischen Säure, wie zum Beispiel Zitronensäure oder Weinsäure nachgespült. Anschließend wird das Haar getrocknet.

Das erfindungsgemäße Oxidationshaarfärbemittel ermöglicht Haarfärbungen mit ausgezeichneten licht-, Wasch- und Reibechtheit. Die sehr guten färberischen Eigenschaften des neuen Mittels zeigen sich besonders darin, daß das Mittel die Anfärbungen von ergrauten, chemisch nicht vorbehandelten Haaren problemlos und mit außergewöhnlicher Deckkraft ermöglicht, obwohl es einen niedrigeren Ammoniakgehalt als übliche Mittel aufweist.

Die nachfolgenden Beispiele sollen den Gegenstand der Erfindung näher erläutern.

## Beispiele

**Beispiel 1:** Haarfärbemittel in Cremeform

```
  3,60 g   2,5-Diaminotoluol-sulfat
  1,80 g   Resorcin
  0,50 g   m-Aminophenol
  0,06 g   2-Amino-6-chlor-4-nitrophenol-
           hydrochlorid
  0,50 g   Ascorbinsäure
  8,00 g   Ammoniak, 25prozentig
 15,00 g   Cetylalkohol
  3,50 g   Natrium-Laurylalkoholdiglykol-
           ethersulfat,
           26prozentig
 67,04 g   Wasser
100,00 g
```

Man vermischt vor dem Gebrauch 50 g dieses Haarfärbemittels mit 50 g einer 6prozentigen Wasserstoffperoxidlösung oder einer Wasserstoffperoxidemulsion und läßt das Gemisch 30 Minuten lang bei 40 Grad Celsius auf ergrautes, menschliches Haar einwirken. Danach wird mit Wasser nachgespült und getrocknet. Das Haar hat eine gleichmäßige dunkelbraune Färbung angenommen. Das erfindungsgemäße Mittel zeichnet sich durch seine außergewöhnliche Deckkraft bei ergrautem menschlichen Haar und seinen im Vergleich zu üblichen Mitteln niedrigen Ammoniakgehalt aus.

**Beispiel 2:** Haarfärbemittel in flüssiger Form

```
   2,50 g   2,5-Diaminotoluol-sulfat
   0,80 g   Resorcin
   0,30 g   m-Aminophenol
   0,30 g   2-Amino-6-chlor-4-nitrophenol-
            hydrochlorid
   0,80 g   3-Amino-2-methylamino-6-methoxy-
            pyridin-Dihydrochlorid
   0,50 g   Ascorbinsäure
  12,00 g   Ölsäure
  15,00 g   Natrium-Laurylalkoholdiglykol-
            ethersulfat, 26prozentig
  18,00 g   Laurylalkoholdiglykoletner
  19,00 g   Ethanol, rein
   8,00 g   Ammoniak, 25prozentig
  22,80 g   Wasser
 100,00 g
```

50 g dieses Haarfärbemittels werden kurz vor dem Gebrauch mit 25 ml Wasserstoffperoxidlösung (6prozentig) vermischt und das Gemisch anschließend auf ergrautes, menschliches Haar aufgetragen. Nach einer Einwirkzeit von 30 Minuten bei Raumtemperatur wird ausgespült und getrocknet. Das Haar ist gleichmäßig dunkelaschbraun gefärbt.

**Beispiel 3:** Haarfärbemittel in flüssiger Form

```
  2,50 g   2-(2,5'-Diaminophenyl)ethanol
  0,80 g   Resorcin
  0,30 g   m-Aminophenol
  0,30 g   2-Amino-6-chlor-4-nitrophenol-
           hydrochlorid
  0,80 g   3-Amino-2-methylamino-6-methoxy-
           pyridin-Dihydrochlorid
  0,50 g   Ascorbinsäure
 12,00 g   Ölsäure
 15,00 g   Natrium-Laurylalkoholdiglykol-
           ethersulfat, 26prozentig
 18,00 g   Laurylalkoholdiglykolether
 19,00 g   Ethanol, rein
  8,00 g   Ammoniak, 25prozentig
 22,80 g   Wasser
100,00 g
```

Man vermischt kurz vor dem Gebrauch 50 g dieses Haarfärbemittels mit 25 ml Wasserstoffperoxidlösung (6prozentig) und läßt das Gemisch anschließend 30 Minuten bei Raumtemperatur auf ergrautes, menschliches Haar einwirken. Demnach wird ausgespült und das Haar getrocknet. Das Haar hat eine gleichmäßige lebhafte dunkelaschbraune Färbung angenommen.

**Beispiel 4:** Haarfärbemittel in Cremeform

```
  3,60 g   2-(2,5'-Diaminophenyl)ethanol-
           hydrochlorid
  1,80 g   Resorcin
  0,50 g   m-Aminophenol
  0,06 g   2-Amino-6-chlor-4-nitrophenol-
           hydrochlorid
  0,50 g   Ascorbinsäure
 15,00 g   Cetylalkohol
  3,50 g   Natrium-Laurylalkoholdiglykol-
           ethersulfat,
           26prozentig
  8,00 g   Ammoniak, 25prozentig
 67,04 g   Wasser
100,00 g
```

Man vermischt kurz vor dem Gebrauch 50 g dieses Haarfärbemittels mit 50 g einer 6prozentigen Wasserstoffperoxidlösung oder einer Wasserstoffperoxidemulsion und läßt das Gemisch 30 Minuten lang bei 40 Grad Celsius auf ergrautes, menschliches Haar einwirken. Danach wird mit Wasser nachgespült und getrocknet. Das Haar hat eine gleichmäßige dunkelbraune Färbung angenommen. Das erfindungsgemäße Mittel zeichnet sich durch seine außergewöhnliche Deckkraft bei ergrautem menschlichen Haar und seinen im Vergleich zu üblichen Mitteln niedrigen Ammoniakgehalt aus. Daneben ist das vorstehende Mittel ausgezeichnet physiologisch verträglich und verursacht keine Sensibilisierungsreaktionen.

**Beispiel 5:** Haarfärbemittel in flüssiger Form

```
   2,50 g   2,5-Diaminotoluol-sulfat
   0,80 g   Resorcin
   0,30 g   m-Aminophenol
   0,30 g   2-Amino-6-chlor-4-nitrophenol-
            hydrochlorid
   0,80 g   3-Amino-2-methylamino-6-methoxy-
            pyridin-Dihydrochlorid
   0,50 g   Ascorbinsäure
  12,00 g   Ölsäure
  15,00 g   Natrium-Laurylalkoholdiglykol-
            ethersulfat, 26prozentig
  18,00 g   Laurylalkoholdiglykolether
  19,00 g   Ethanol, rein
   2,00 g   Ammoniak, 25prozentig
  28,80 g   Wasser
 100,00 g
```

50 g dieses Haarfärbemittels werden kurz vor dem Gebrauch mit 25 ml Wasserstoffperoxidlösung (6prozentig) vermischt und das Gemisch anschließend auf ergrautes, menschliches Haar aufgetragen. Nach einer Einwirkzeit von 30 Minuten bei Raumtemperatur wird ausgespült und getrocknet. Das Haar ist gleichmäßig dunkelaschbraun gefärbt.

Alle in der vorliegenden Anmeldung angegebenen Prozentzahlen stellen Gewichtsprozent dar.

**Patentansprüche**

1.  Mittel zur oxidativen Färbung von Haaren auf der Basis einer in der Haarfärbung üblichen Entwicklersubstanz-Kupplersubstanz-Kombination, dadurch gekennzeichnet, daß es 0,01 bis 1,0 Gewichtsprozent 2-Amino-6-chlor-4-nitrophenol oder dessen physiologisch verträgliches, wasserlösliches Salz enthält.

2.  Mittel nach Anspruch 1, dadurch gekennzeichnet, daß es 0,05 bis 0,3 Gewichtsprozent 2-Amino-6-chlor-4-nitrophenol enthält.

3.  Mittel nach Anspruch 1 oder 2, dadurch gekennzeichnet, daß die Kuppler- und Entwicklersubstanzen ausgewählt sind aus Resorcin, 4-Chlorresorcin, 4,6-Dichlorresorcin, 2-Methylresorcin, 2-Amino-4-(2'-hydroxyethylamino)-anisol, 2,4-Diaminobenzylalkohol, m-Phenylendiamin, 5-Amino-2-methylphenol, 2,4-Diaminophenoxyethanol, 1-Naphthol, m-Aminophenol, 3-Amino-4-chlor-6-methylphenol, 3-Amino-2-methylphenol, 4-Amino-2-hydroxyphenoxyethanol, 4-Hydroxy-1,2-methylendioxybenzol, 4-(2'-Hydroxyethylamino)-1,2-methylendioxybenzol, 2,4-Diamino-5-ethoxytoluol, 4-Hydroxyindol, 3-Amino-5-hydroxy-2,6-dimethoxypyridin, 3,5-Diamino-2,6-dimethoxypyridin, 2,6-Dimethoxypyridin, 2,5-Diaminotoluol, 2,5-Diaminobenzylalkohol, 2-(2,5'-Diaminophenyl)ethanol, 4-Aminophenol, 4-Amino-3-methylphenol, 4-Amino-2-methoxymethylphenol, 4-Amino-2-ethoxymethylphenol sowie Tetraaminopyrimidin oder deren physiologisch verträglichen, wasserlöslichen Salzen.

4. Mittel nach einem der Ansprüche 1 oder 2, dadurch gekennzeichnet, daß es als Entwicklersubstanz 2-(2',5'-Diaminophenyl)ethanol oder dessen physiologisch verträgliches Salz und mindestens eine Kupplersubstanz, die ausgewählt ist aus Resorcin, 2-Methylresorcin, 4-Chlorresorcin, 3,4-Methylendioxyphenol, m-Aminophenol und N-(2'-Hydroxyethyl)-3,4-methylendioxyanilin oder deren physiologisch verträglichen, wasserlöslichen Salzen, enthält.

5. Mittel nach einem der Ansprüche 1 bis 4, dadurch gekennzeichnet, daß die Entwicklersubstanz-Kupplersubstanz-Kombination in einer Menge von 0,01 bis 6,0 Gewichtsprozent enthalten ist.

6. Mittel nach einem der Ansprüche 1 bis 5, dadurch gekennzeichnet, daß es mindestens einen der direktfärbenden Farbstoffe Diamond Fuchsin (C.I. 42510), Leather Ruby HF (C.I. 42520), 2-Amino-4,6-dinitrobenzol, 2-Amino-5-(2'-hydroxyethylamino)-nitrobenzol, 4-(2'-Hydroxyethylamino)-3-nitrotoluol, 1-(2'-Ureidoethylamino)-4-nitrobenzol, 2-Amino-5-nitrophenol, Acid Brown 4 (C.I. 14805), 1,4-Diaminoanthrachinon und 1,4,5,8-Tetraaminoanthrachinon enthält.

7. Mittel nach einem der Ansprüche 1 bis 6, dadurch gekennzeichnet, daß es als Antioxidantien Ascorbinsäure, Thioglykolsäure oder Natriumsulfit enthält.

8. Mittel nach einem der Ansprüche 1 bis 7, dadurch gekennzeichnet, daß es einen pH-Wert von 8 bis 11,5 aufweist.

9. Mittel nach einem der Ansprüche 1 bis 8, dadurch gekennzeichnet, daß es 0,01 bis 2,50 Gewichtsprozent Ammoniak enthält.

10. Verfahren zur oxidativen Färbung von Haaren, dadurch gekennzeichnet, daß man ein Oxidationshaarfärbemittel gemäß einem der Ansprüche 1 bis 9 kurz vor dem Gebrauch mit einem Oxidationsmittel mischt, 60 bis 200 g dieses Gemischs auf die Haare aufbringt, sodann das Mittel 10 bis 45 Minuten lang bei einer Temperatur von 15 bis 50 Grad Celsius einwirken läßt, anschließend die Haare mit Wasser spült und sodann trocknet.


**Claims**

1. Agent for the oxidative dyeing of hair based on a developer substance - coupler substance combination conventional in hair dyeing, characterised in that it contains 0.01 to 1.0 weight % of 2-amino-6-chloro-4-nitrophenol or its physiologically compatible water-soluble salt.

2. Agent according to Claim 1, characterised in that it contains 0.05 to 0.3 weight % of 2-amino-6-chloro-4-nitrophenol.

3. Agent according to Claim 1 or 2, characterised in that the coupler and developer substances are selected from resorcinol, 4-chlororesorcinol, 4,6-dichlororesorcinol, 2-methylresorcinol, 2-amino-4-(2'-hydroxyethylamino)-anisole, 2,4-diaminobenzyl alcohol, m-phenylenediamine, 5-amino-2-methylphenol, 2,4-diaminophenoxyethanol, 1-naphthol, m-aminophenol, 3-amino-4-chloro-6-methylphenol, 3-amino-2-methylphenol, 4-amino-2-hydroxyphenoxyethanol, 4-hydroxy-1,2-methylenedioxybenzene, 4-(2'-hydroxyethylamino)-1,2-methylenedioxybenzene, 2,4-diamino-5-ethoxytoluene, 4-hydroxyindole, 3-amino-5-hydroxy-2,6-dimethoxypyridine, 3,5-diamino-2,6-dimethoxypyridine, 2,6-dimethoxypyridine, 2,5-diaminotoluene, 2,5-diaminobenzyl alcohol, 2-(2',5'-diaminophenyl)ethanol, 4-amino-phenol, 4-amino-3-methylphenol, 4-amino-2-methoxymethylphenol, 4-amino-2-ethoxymethylphenol and tetraaminopyrimidine or their physiologically compatible water-soluble salts.

4. Agent according to either of Claims 1 or 2, characterised in that it contains as developer substance 2-(2',5'-diaminophenyl)ethanol or its physiologically compatible salt and at least one coupler substance which is selected from the group comprising resorcinol, 2-methylresorcinol, 4-chlororesorcinol, 3,4-methylenedioxyphenol, m-aminophenol and N-(2'-hydroxyethyl)-3,4-methylenedioxyaniline or their physiologically compatible water-soluble salts.

5. Agent according to any one of Claims 1 to 4, characterised in that the developer substance - coupler substance combination is present in an amount from 0.01 to 6.0 weight %.

6. Agent according to any one of Claims 1 to 5, characterised in that it contains at least one of the direct-acting dyes Diamond Fuchsin (C.I. 42510), Leather ruby HF (C.I. 42520), 2-amino-4,6-dinitrobenzene, 2-amino-5-(2'-hydroxyethylamino)-nitrobenzene, 4-(2'-hydroxyethylamino)-3-nitrotoluene, 1-(2'-ureidoethylamino)-4-nitrobenzene, 2-amino-5-nitrophenol, Acid Brown 4 (C.I. 14805), 1,4-diaminoanthraquinone and 1,4,5,8-tetraaminoanthraquinone.

7. Agent according to any one of Claims 1 to 6, characterised in that it contains ascorbic acid, thioglycolic acid or sodium sulphite as anti-oxidants.

8. Agent according to any one of Claims 1 to 7, characterised in that it has a pH of 8 to 11.5.

9. Agent according to any one of Claims 1 to 8, characterised in that it contains 0.01 to 2.50 weight % of ammonia.

10. Process for the oxidative dyeing of hair, characterised in that an oxidative hair dye according to any one of Claims 1 to 9 is mixed with an oxidising agent shortly before use, 60 to 200 grams of this mixture is applied to the hair, the agent is then left to react for 10 to 45 minutes at a temperature of 15 to 50°C, the hair is then rinsed with water and subsequently dried.


**Revendications**

1. Produit de teinture par oxydation des cheveux, à base d'une combinaison, usuelle dans la teinture des cheveux, substance révélateur-substance de couplage, caractérisé en ce que le produit contient de 0,01 à 1,0 pourcent en poids de 2-amino-6-chlor-4-nitrophénol ou de son sel physiologiquement compatible, soluble dans l'eau.

2. Produit selon la revendication 1, caractérisé en ce qu'il contient de 0,05 à 0,3 pourcent en poids de 2-amino-6-chlor-4-nitrophénol.

3. Produit selon la revendication 1 ou 2, caractérisé en ce que les substances de couplage et révélateur sont sélectionnées parmi la résorcine, la 4-chlororésorcine, 4,6-di-chlororésorcine, la 2-méthylrésorcine, la 2-amino-4-(2'-hydroxyéthylamino)-anisol, 2,4-diaminobenzylalcool, m-phénylènediamine, 5-amino-2-méthylphénol, 2,4-diaminophénoxyéthanol, 1-naphthol, m-aminophénol, 3-amino-4-chloro-6-méthylphénol, 3-amino-2-méthylphénol, 4-amino-2-hydroxyphénoxyéthanol, 4-hydroxy-1,2-méthylène-dioxybenzène, 4-(2'-hydroxyéthylamino)-1,2-méthylène-dioxybenzène, 2,4-diamino-5-éhoxytoluène, 4-hydroxyindol, 3-amino-5-hydroxy-2,6-diméthoxypyridine, 3,5-diamino-2,6-diméthoxypyridine, 2,6-diméthoxypyridine, 2,5-diaminotoluène, 2,5-diaminobenzylalcool, 2-(2,5'-diaminophényl)éthanol, 4-aminophénol, 4-amino-3-méthylphénol, 4-amino-2-méthoxyméthylphénol, 4-amino-2-éthoxyméthylphénol, ainsi que tétyraaminopyridine ou leurs sels physiologiquement compatibles, solubles dans l'eau.

4. Produit selon l'une des revendications 1 ou 2, caractérisé en ce qu'il contient comme substance révélateur du 2-(2',5'-diaminophényl)éthanol ou son sel physiologiquement compatible et au moins une substance de couplage, qui est sélectionnée parmi la résorcine, la 2-méthylrésorcine, la 4-chlororésorcine, 3,4-méthylènedioxyphénol, m-aminophénol et N-(2'-hydroxyéthyl-3,4-méthylènedioxyaniline, ou leurs sels physiologiquement compatibles, solubles dans l'eau.

5. Produit selon l'une des revendications 1 à 4, caractérisé en ce que la combinaison substance révélateur-substance de couplage est contenue en une quantité allant de 0,01 à 6,0 pourcent en poids.

6. Produit selon l'une des revendications 1 à 5, caractérisé en ce qu'il contient au moins l'un des colorants à action colorante directe que sont le Diamond Fuchsin (C.I.42510), Leather Ruby HF (C.I.42520), 2-amino-4,6-dinitrobenzène, 2-amino-5-(2'-hydroxyéthylamino)-nitrobenzène, 4-(2'-hydroxyéthylamino)-3-nitrotoluène, 1-(2'-uréidoéthylamino)-4-nitrobenzène, 2-amino-5-nitrophénol, Acid Brown 4 (C.I. 14805), 1,4-diaminoantrachinone et 1,4,5,8-tétraminoanthrachinone.

7. Produit selon l'une des revendications 1 à 6 caractérisé en ce qu'il contient comme antioxydant l'acide ascorbique, l'acide thioglycolique ou du sulfite de sodium.

8. Produit selon l'une des revendications 1 à 7, caractérisé en ce qu'il présente un pH allant de 8 à 11,5.

9. Produit selon l'une des revendications 1 à 8, caractérisé en ce qu'il contient de 0,01 à 2,50 pourcent en poids d'ammoniac.

10. Procédé de teinture par oxydation des cheveux, caractérisé en ce qu'on mélange un produit de teinture oxydant des cheveux selon l'une des revendications 1 à 9, peu avant l'utilisation avec un produit oxydant, on applique de 60 à 200 g de ce mélange sur les cheveux, on laisse ensuite agir le produit de 10 à 45 minutes à une température de 15 à 50 degrés Celsius, on rince ensuite les cheveux à l'eau puis on les sèche.